(19) Europäisches Patentamt
European Patent Office
Office européen des brevets

(11) Veröffentlichungsnummer: **0 173 244**
**B1**

(12) # EUROPÄISCHE PATENTSCHRIFT

(45) Veröffentlichungstag der Patentschrifft: **07.02.90**

(21) Anmeldenummer: **85110519.7**

(22) Anmeldetag: **21.08.85**

(51) Int. Cl. ⁵: **C 07 C 275/54**, C 07 D 295/12, C 07 C 211/52, C 07 C 273/18, A 01 N 47/34

(54) **Benzoylphenylharnstoffe.**

(30) Priorität: **24.08.84 CH 4042/84**

(43) Veröffentlichungstag der Anmeldung: **05.03.86 Patentblatt 86/10**

(45) Bekanntmachung des Hinweises auf die Patenterteilung: **07.02.90 Patentblatt 90/06**

(84) Bennante Vertragsstaaten: **AT BE CH DE FR IT LI NL**

(56) Entgegenhaltungen: **EP-A-0 065 487**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Drabek, Jozef, Dr.**
**Benkenstrasse 12**
**D-4104 Oberwil (CH)**
Erfinder: **Böger, Manfred**
**Wilhelm Glockstrasse 14**
**D-7858 Weil am Rhein 45 (DE)**
Erfinder: **Kristiansen, Odd, Dr.**
**Delligrabenstrasse 7**
**CH-4313 Möhlin (CH)**

(74) Vertreter: **Zumstein, Fritz, Dr.**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBERGRAF, STOCKHOLM 1990

**Beschreibung**

Die vorliegende Erfindung betrifft neue substituierte Benzoyl-amino-phenylharnstoffe, Verfahren und Zwischenprodukte zu ihrer Herstellung und ihre Verwendung in der Schädlingsbekämpfung.

Die erfindungsgemässen Verbindungen haben die Formel I

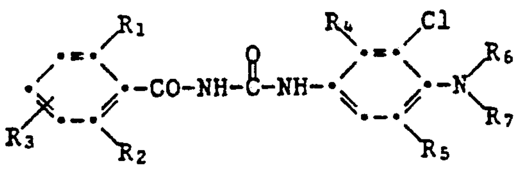

(I),

worin

$R_1$ Wasserstoff, Halogen, $CF_3$ oder $C_1$-$C_4$-Alkoxy;
$R_2$ Halogen, $C_1$-$C_4$-Alkyl, $CF_3$ oder $C_1$-$C_4$-Alkoxy;
$R_3$ Wasserstoff, Halogen, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_6$-Alkyl, $C_3$-$C_5$-Alkenyl, $C_3$-$C_5$-Alkynyl oder mit 1 bis 8 Fluor- oder Chloratomen, Methoxy oder Äthoxy substituiertes $C_1$-$C_4$-Alkyl; oder
$R_6$ und $R_7$ zusammen mit dem N-Atom, an das sie gebunden sind, den

Rest 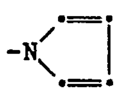 ;

bedeuten, wobei der Ausdruck "Halogen" für Fluor, Chlor oder Brom steht.

Erfindungsgemäss bevorzugt werden Verbindungen der Formel I, worin

$R_1$ Wasserstoff, Halogen, $CF_3$ oder $C_1$-$C_4$-Alkoxy;
$R_2$ Halogen, $C_1$-$C_4$-Alkyl, $CF_3$ oder $C_1$-$C_4$-Alkoxy;
$R_3$ Wasserstoff, Halogen, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_6$-Alkyl, $C_3$-$C_5$-Alkenyl, $C_3$-$C_5$-Alkynyl oder mit 1 bis 8 Fluor- oder Chloratomen, Methoxy oder Äthoxy substituiertes $C_1$-$C_4$-Alkyl;
bedeuten, wobei der Ausdruck "Halogen" für Fluor, Chlor oder Brom steht.

Hervorzuheben sind diejenigen Verbindungen der Formel I, worin

$R_1$ Wasserstoff, Fluor, Chlor, $CF_3$, Methoxy oder Äthoxy;
$R_2$ Fluor, Chlor, $C_1$-$C_3$-Alkyl, $CF_3$ oder Methoxy;
$R_3$ Wasserstoff, Fluor. Chlor, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_4$-Alkyl, Alkyl, Propargyl oder mit 1 bis 7 Fluor- oder Chloratomen oder Methoxy substituiertes $C_1$-$C_3$-Alkyl;
bedeuten.

Hervorzuheben sind ferner diejenigen Verbindungen der Formel I, worin

$R_1$ Wasserstoff, Fluor, Chlor oder Methoxy;
$R_2$ Fluor, Chlor, Methyl oder Methoxy;
$R_3$ Wasserstoff oder Fluor;
$R_4$ Fluor oder Chlor;
$R_5$ Wasserstoff oder Chlor;
$R_6$ und $R_7$ $C_1$-$C_4$-Alkyl;
bedeuten.

Weiterhin bevorzugte Verbindungen der Formel I sind dadurch gekennzeichnet, dass $R_6$ Methyl und $R_7$ $C_1$-

$C_4$-Alkyl und dadurch gekennzeichnet, dass $R_4$ Fluor und $R_5$ Wasserstoff oder Chlor bedeuten.

Unter dem Begriff Alkyl im Rahmen der vorliegenden Erfindung sind geradkettige und verzweigte Alkylreste und je nach Zahl der angegebenen Kohlenstoffatome beispielsweise folgende Gruppen zu verstehen: Methyl, Äthyl, Propyl, Butyl und Pentyl, sowie ihre Isomeren, wie z. B. n-Propyl, Isopropyl, Isobutyl, tert.-Butyl, Isopentyl usw.

Unter dem Begriff Halogen im Rahmen der vorliegenden Erfindung ist Fluor, Chlor und Brom zu verstehen, vorzugsweise Fluor und Chlor.

Die Verbindungen der Formel I können analog an sich bekannter Verfahren hergestellt werden (vgl. u. a. die deutschen Offenlegungsschriften Nr. 2 123 236, 2 601 780 und 3 240 975).

So kann man z. B. eine Verbindung der Formel I erhalten, durch Umsetzung

a) einer Verbindung der Formel II

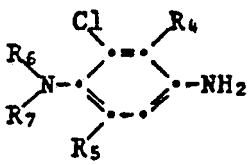

(II)

mit einer Verbindung der Formel III

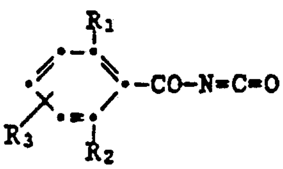

(III)

oder

b) einer Verbindung der Formel IV

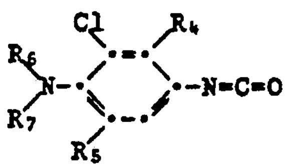

(IV)

gegebenenfalls in Gegenwart einer organischen oder anorganischen Base mit einer Verbindung der Formel V

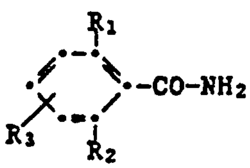

(V),

oder

c) einer Verbindung der Formel II mit einer Verbindung der Formel VI

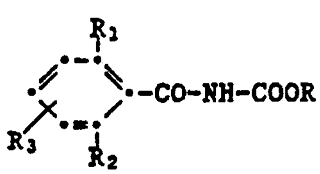

(VI).

In den obigen Formeln II bis VI haben die Reste $R_1$ bis $R_7$ die unter Formel I vorstehend angegebenen Bedeutungen, und R bedeutet einen $C_1$-$C_8$-Alkylrest, der gegebenenfalls mit Halogen, vorzugsweise Chlor, substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen

sich z. B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone. z. B. Aceton, Methyläthylketon, Methylisopropylketon und Methylisobutylketon. Verfahren a) wird im allgemeinen bei einer Temperatur von -10 bis 200°C, vorzugsweise zwischen 50 und 150°C, gegebenenfalls in Gegenwart einer organischen Base, z. B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d. h. für die Umsetzung der Urethane der Formel VI mit einem Phenylendiamin der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III und V sind bekannt und können analog bekannten Verfahren hergestellt werden. Bei den Ausgangsstoffen der Formeln II handelt es sich um neuartige Verbindungen, die ebenfalls einen Gegenstand der vorliegenden Erfindung bilden. Die Verbindungen der Formel II können in an sich bekannter Weise dadurch hergestellt werden, dass man entsprechend substituierte p-Nitraniline der Formel VII

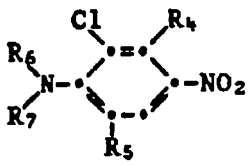

(VII)

in Analogie zu dem in J. Org. Chem. 29 (1964), 1, aufgezeigten Verfahren hydriert (vgl. auch die dort zitierte Literatur). Aber auch durch chemische Reduktion (z. B. mittels Sn-(II)-Chlorid/HCl) der entsprechenden Nitroverbindungen der Formel VII sind Phenylendiamine der Formel II zugänglich (vgl. Houben Weyl, "Methoden d. org. Chemie" 11/1, 422). Die Verbindungen der Formel VII sind zugänglich durch N-Alkylierung entsprechender Nitraniline mit unsubstituierter Aminogruppe oder durch Umsetzung entsprechender 4-Halogennitrobenzole mit den erwünschten Dialkylaminen. 4-Amino-phenylisocyanate der Formel IV können z. B. hergestellt werden durch Phosgenierung von p-Phenylendiaminen der Formel II nach allgemein üblichen Arbeitsweisen.

Zu Benzoylisocyanaten gemäss Formel III kann man unter anderem wie folgt gelangen (vgl. J. Agr. Food Chem. 21, 348 und 993; 1973):

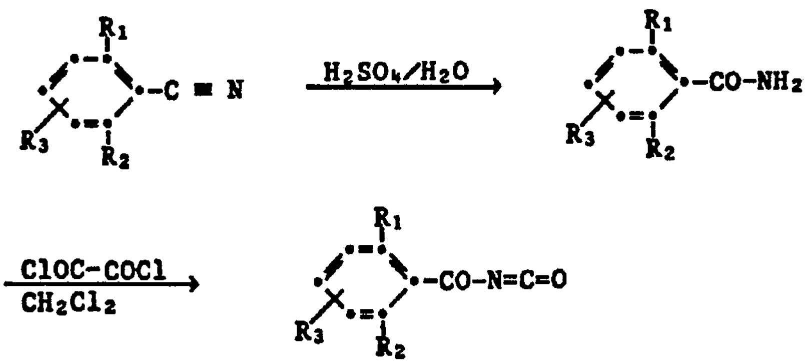


(III)

Die 4-Amino-phenylisocyanate der Formel IV lassen sich z. B. durch Phosgenierung der Aniline der Formel II nach allgemein üblichen Verfahren herstellen. Die weiterhin als Ausgangsstoffe zu verwendenden Benzamide der Formel V sind bekannt (vgl. z. B. Beilstein "Handbuch der organischen Chemie" Bd. 9, S. 336).

Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure.

Aus den deutschen Offenlegungsschriften Nr. 3 233 341 und 3 233 383 sowie den europäischen Patentanmeldungen Nr. 0 016 729, 0 055 213, 0 065 487 und 0 072 438 sind substituierte N-Benzoyl-N'-(4-amino-phenyl)-harnstoffe mit pestizider Wirksamkeit bekannt. Hierbei handelt es sich im wesentlichen um Verbindungen, deren am N'-Stickstoff gebundene Phenylgruppe nur in 3- und 5-Stellung mit Halogen, $CF_3$ oder Methyl substituiert ist und welche in der 4-Stellung eine mit Alkyl, Alkenyl, Alkynyl oder Phenyl substituierte Aminogruppe aufweist. Ferner sind aus J. Agr. Food Chem. 21, No. 3, Seite 353 (1973) N-2,6-Dichlorbenzoyl-N'-(4-dimethylamino)-phenyl- und -N'-(3-Chlor-4-dimethylamino)-phenyl-harnstoffe bekannt, die jedoch - wie aus der dort aufgeführ-

ten Tabelle III ersichtlich - nur unzureichende Insektizidwirkung zeigen.

Gegenüber diesen bekannten Stoffgruppen weisen die erfindungsgemässen neuen Verbindungen der Formel I als charakteristisches Strukturmerkmal einen obligaten Substituenten in 2-Stellung am N'-ständigen Phenylring auf. Überraschenderweise wurde gefunden, dass die vorliegenden Verbindungen bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z. B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z. B. Spodoptera littoralis und Heliothis virescens), sowie in Obst- und Gemüsekulturen (z. B. Laspeyresia pomonella, Leptinotarsa decemlineata und Epilachna varivestis) eingesetzt werden. Die Verbindungen der Formel I zeichnen sich durch eine ausgeprägte ovizide und vor allem larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z. B. durch Tier-, Stall- und Weidebehandlung.

Die Verbindungen der Formel I eignen sich ferner zur Bekämpfung der folgenden Obst- und Gemüsekulturen befallenden Milbenspezies: Tetranychus urticae, Tetranychus cinnabarinus, Panonychus ulmi, Broybia rubrioculus, Panonychus citri, Eriophyes piri, Eriophyes ribis, Eriophyes vitis, Tarsonemus pallidus, Phyllocoptes vitis und Phyllocoptruta oleivora.

Die gute pestizide Wirkung der erfindungsgemässen Verbindungen der Formel I entspricht einer Abtötungsrate (Mortalität) von mindestens 50 - 60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z. B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d. h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Äther und Ester, wie Äthanol, Äthylenglykol, Äthylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnußöl oder Sojaöl, oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger-kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z. B. die Na- oder K-Salze der Öl oder Stearinsäure, oder von natürlichen Fettsäuregemischen, die z. B. aus Kokosnuss- oder Talgöl gewonnen werden können. Ferner sind als Tenside auch die

Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z. B. das Na- oder CaSalz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit etwa 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z. B. Salze des Phosphorsäureesters eines p-Nonylphenol-(4-14)-Äthylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykolüthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Äthylenglykoläthergruppen und 10 bis 100 Propylenglykoläthergruppen enthaltenden Polyäthylenoxid-Addukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyl-di-(2-chloräthyl)-äthylammoniumbromid.

Die in der Formulierungstechnik gebräuchlichen Tenside sind u. a. in folgenden Publikationen beschrieben:

"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Beispiel 1**

**a) Herstellung von 2-Fluor-3,5-dichlor-4-(N-methyl-N-n-butylamino)-nitrobenzol**

22,8 g 2,4-Difluor-3,5-dichlornitrobenzol werden in 100 ml Dimethylsulfoxid gelöst. Unter Rühren werden zu dieser Lösung 17.45 g N-Methyl-N-n-butylamin eingetropft. Das Reaktionsgemisch wird dann während 5 Stunden bei Raumtemperatur gerührt. Nach dieser Zeit wird das Lösungsmittel abdestilliert. Der Rückstand wird in Hexan aufgenommen, die Hexan-Lösung mit Wasser gewaschen, getrocknet und das Hexan abdestilliert. Das erhaltene Rohprodukt wird chromatographiert (Säule: 1 m, ø 5 cm; Füllung: Kieselgel; Elutionsmittel: Hexan + 1 % Äther; Fraktionen: à 30 ml). Die gaschromatographische und NMR-Analyse zeigt, dass die Fraktionen Nr. 16 - 57 das gewünschte Produkt in 98 %-iger Reinheit enthalten. Die erhaltene Titelverbindung der Formel

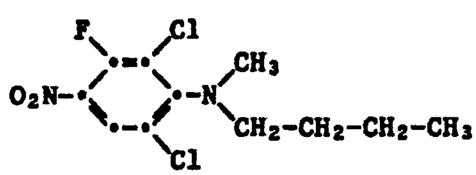

stellt eine gelbliche Flüssigkeit ($n^{22}_D$ = 1,5618) dar.

In entsprechender Weise werden auch die folgenden Nitraniline erhalten:

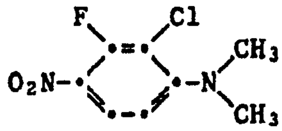

(Smp.: 107 - 108°C)

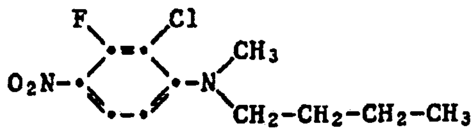

($n^{23}_D$ = 1,6003)

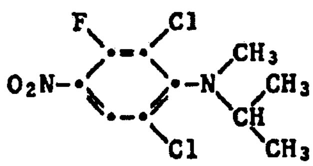

(Smp.: 58 - 61°C)

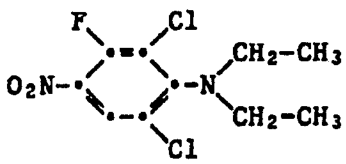

(Kp. 111 - 119°C/1.33 Pa)

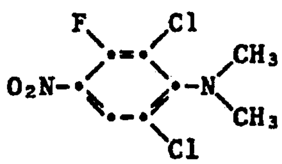

(Smp.: 62 - 66°C)

**b) Herstellung des Ausgangsproduktes 2-Fluor-3,5-dichlor-4-(N-methyl-N-n-butylamino)-anilin**

Es werden 49,2 g des gemäss a) hergestellten 2-Fluor-3,5-dichlor-4-(N-methyl-N-butylamino)-nitrobenzols in 500 ml Tetrahydrofuran unter Zusatz von 10 g und nochmals 5 g Raney-Nickel bei Raumtemperatur unter normalem Druck mit Wasserstoff reduziert. Es wurden 11 Liter Wasserstoff aufgenommen (98 % der Theorie). Nach der Hydrierung wird das Reaktionsgemisch filtriert und aus dem Filtrat das Lösungsmittel abdestilliert. Der Rückstand wird dann am Hochvakuum destilliert. Als blassgelbes Öl (Kp. 102 - 108°C/8 Pa) wird die Titelverbindung der Formel

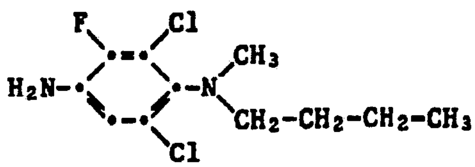

erhalten.

In entsprechender Weise werden die Phenylendiamine der Formeln

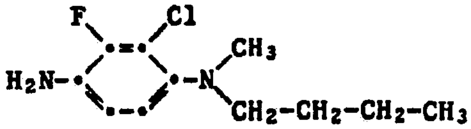

(Kp. 119 - 120°C/6.66 Pa)

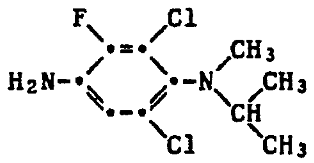

(Kp. 102°C/6.66 Pa)

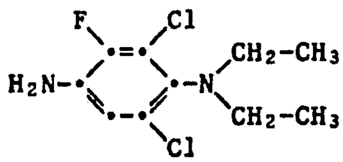

(Kp. 95 - 98°C/80.0 Pa)

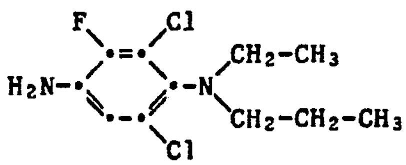

(Kp. 98 - 104°C/0,66 Pa)

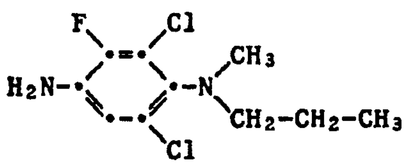

(Kp. 98 - 102°C/9.33 Pa)

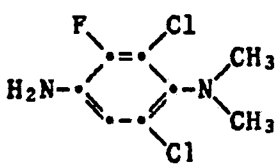

(Kp. 86 - 90°C/12.0 Pa)

hergestellt.

Die vorstehend genannten Verbindungen 2-Fluor-3,5-dichlor-4-(N-methyl-N-n-propylamino)-anilin und 2-Fluor-3,5-dichlor-4-(N-äthyl-N-n-propylamino)-anilin sind auch durch Reduktion von 2-Fluor-3,5-dichlor-4-(N-methyl-N-allyl)-anilin bzw. von 2-Fluor-3,5-dichlor-4-(N-äthyl-N-allyl)-anilin erhältlich.

**c) Herstellung von N-(2,6-Difluorbenzoyl)-N'-[2-fluor-3,5-dichlor-4-(N-methyl N-n-butyl)-aminophenyl]-harnstoff:**

5,3 g 2-Fluor-3,5-dichlor-4-(N-methyl-N-n-butyl)-aminoanilin werden in 50 ml Toluol gelöst. Zu dieser Lösung werden dann 0,05 g Triäthylamin zugegeben. Unter Rühren und Kühlen (Temperatur 0 - 10°C) werden in diese Lösung 3,66 g 2,6-Difluorbenzoylisocyanat eingetropft. Das Reaktionsgemisch wird danach während 10 Stunden bei Rückflusstemperatur gerührt. Das Toluol wird abdestilliert und der Rückstand in Methylenchlorid aufgenommen. Nach Zugabe von Hexan fällt die Titelverbindung der Formel

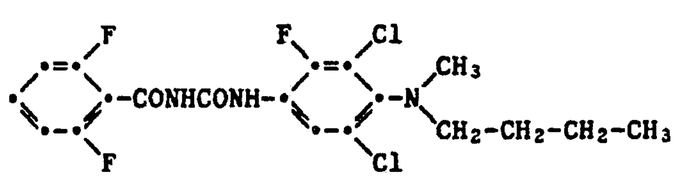

in Form farbloser Kristalle (Smp. 130 - 133°C) aus (Verbindung Nr. 1).

In entsprechender Weise wie vorstehend beschrieben werden auch die folgenden Verbindungen der Formel I hergestellt:

| Verbindung Nr. | | Smp. [°C] |
| --- | --- | --- |
| 2 | 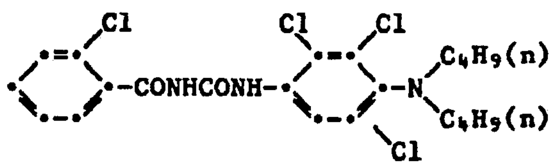 | 185 - 187 |
| 3 | 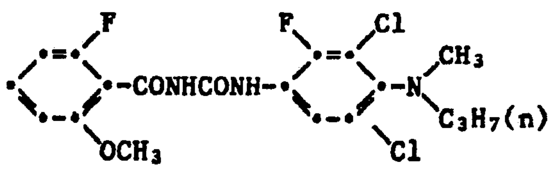 | 105 |
| 4 | 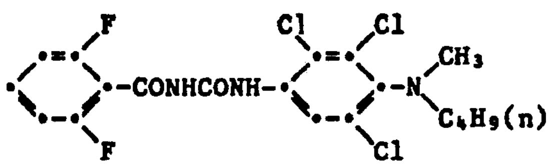 | 134 - 137 |
| 5 | 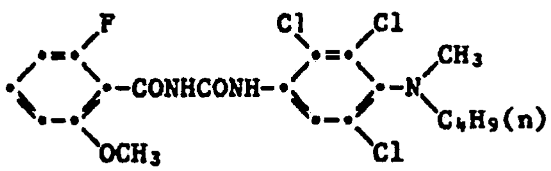 | 148 - 152 |
| 6 | 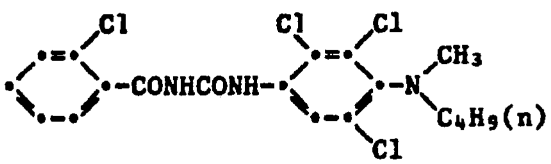 | 117 - 118 |
| 7 | 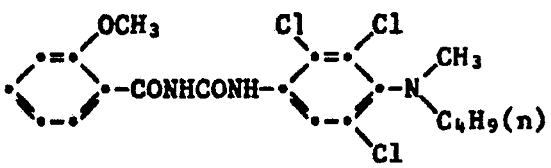 | 137 - 141 |
| 8 | 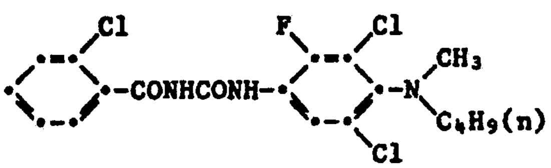 | 114 - 116 |
| 9 | 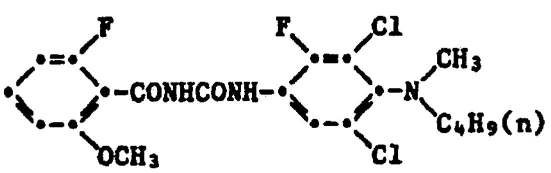 | 104 - 106 |

| Verbindung Nr. | | Smp. [°C] |
|---|---|---|
| 10 | 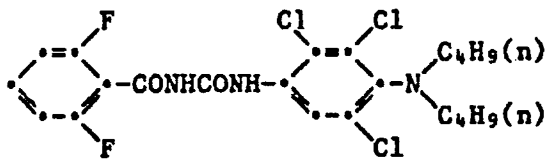 | 190 - 194 |
| 11 | 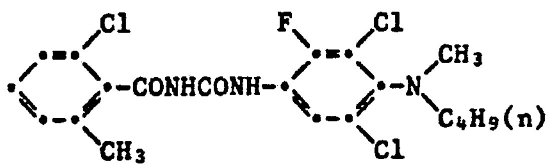 | 125 - 129 |
| 12 | 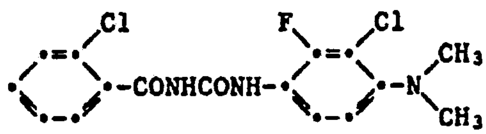 | 202 - 204 |
| 13 | 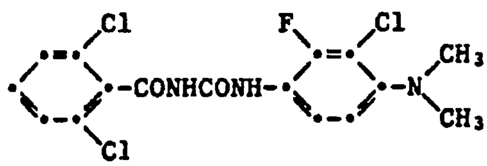 | 185 - 186 |
| 14 | 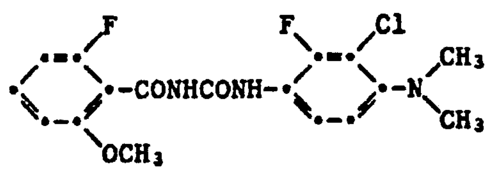 | 145 - 147 |
| 15 | 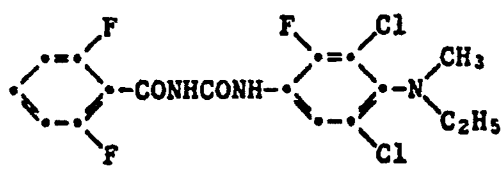 | 165 - 167 |
| 16 | 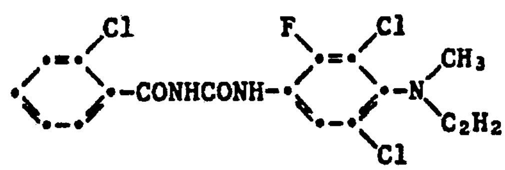 | 152 - 156 |
| 17 | 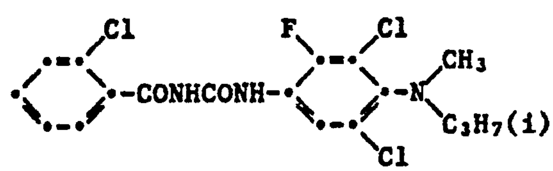 | 162 - 164 |

| Verbindung Nr. | | Smp. [°C] |
|---|---|---|
| 18 | 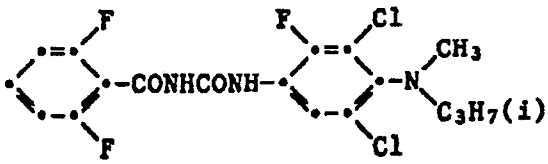 | 171 - 174 |
| 19 | 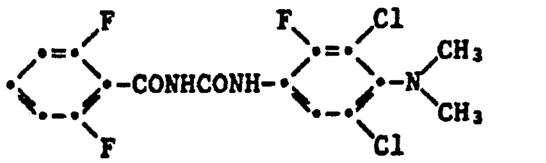 | 195 |
| 20 | 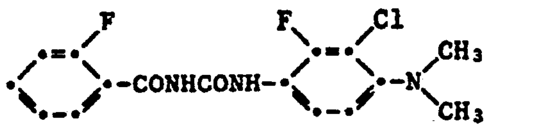 | 194 - 195 |
| 21 | 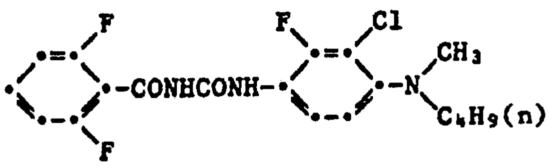 | 130 - 131 |
| 22 | 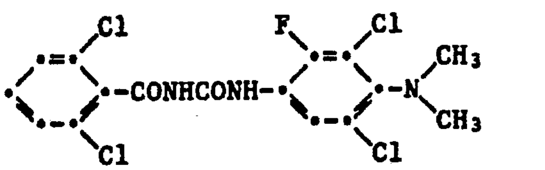 | 226 - 228 |
| 23 | 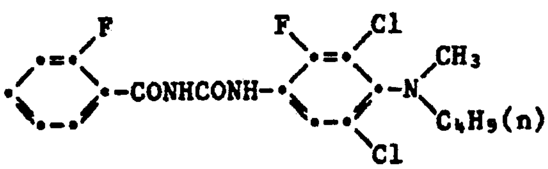 | 90 - 93 |
| 24 | 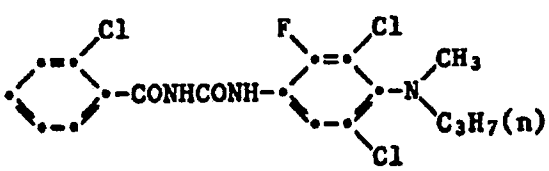 | 107 - 108 |
| 25 | 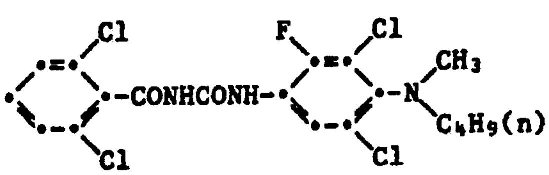 | 129 - 131 |

| Verbindung Nr. | | Smp. [°C] |
|---|---|---|
| 26 | 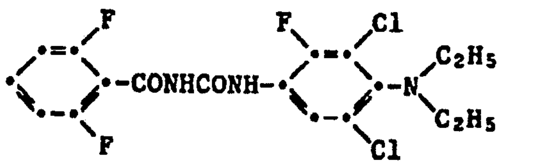 | 155 - 159 |
| 27 | 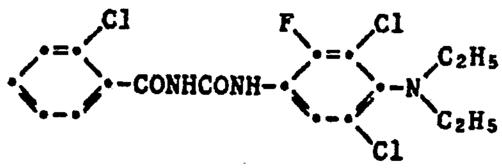 | 144 - 145 |
| 28 | 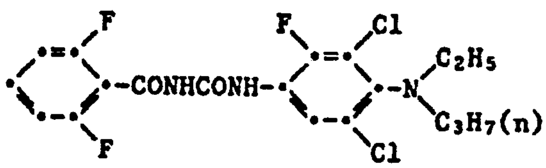 | 136 - 138 |
| 29 | 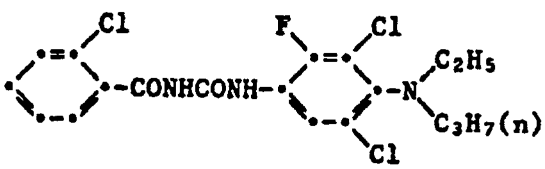 | 135 - 137 |
| 30 | 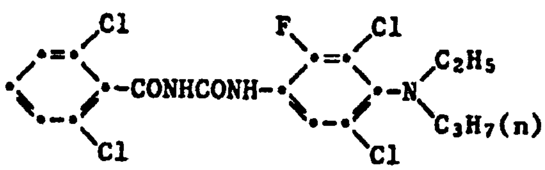 | 136 - 138 |
| 31 | 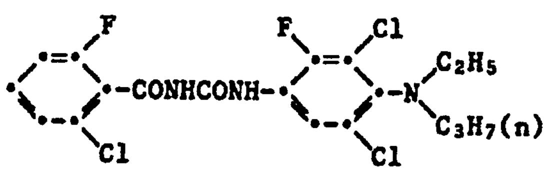 | 135 - 138 |
| 32 | 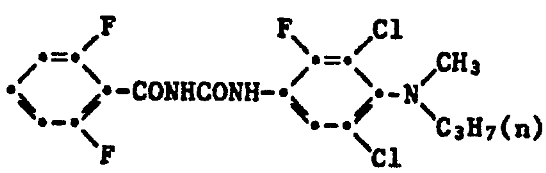 | 127 - 128 |
| 33 | 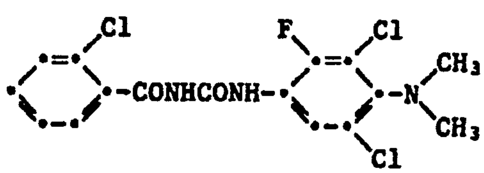 | 188 - 189 |

| Verbindung Nr. | | Smp. [°C] |
|---|---|---|
| 34 | 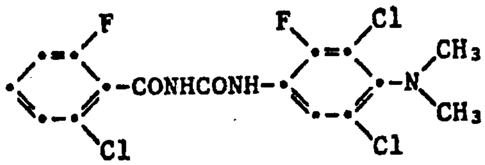 | 214 - 215 |
| 35 | 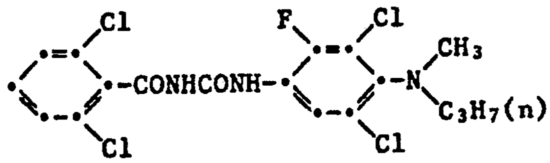 | 101 - 102 |
| 36 | 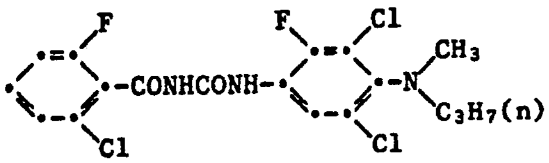 | 116 - 117 |
| 37 | 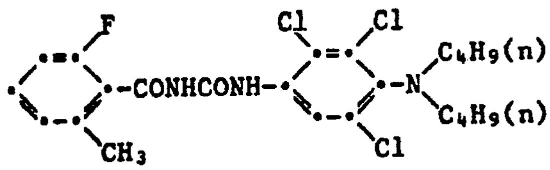 | 189 - 194 |
| 38 | 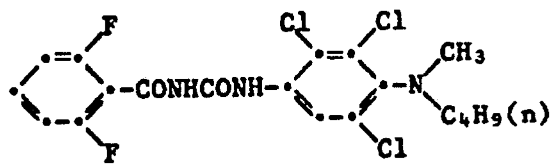 | 134 - 137 |
| 39 | 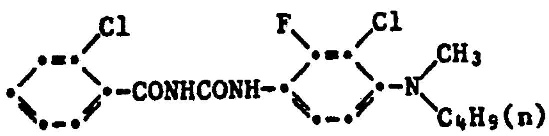 | 145 - 146 |
| 40 | 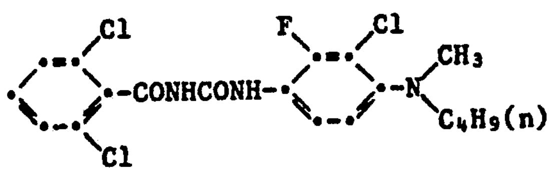 | 144 - 145 |
| 41 | 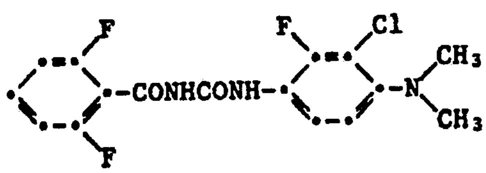 | 195 - 197 |

In entsprechender Weise wie vorstehend beschrieben sind auch die folgenden Verbindungen der Formel I herstellbar:

Verbindung
Nr.

42

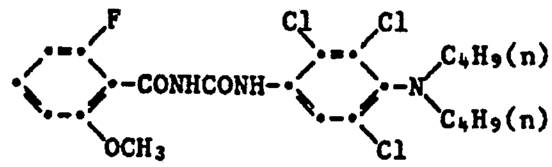

43

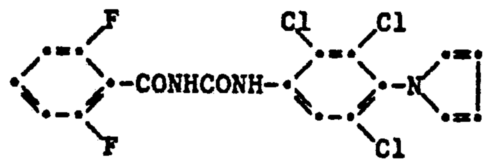

44

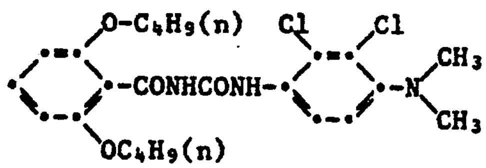

45

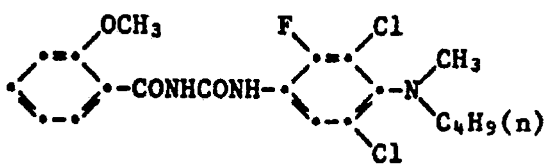

**Beispiel 2**

**Wirkung gegen Musca domestica**

Je 50 g frisch zubereitetes Nährsubstrat für Maden werden in Becher eingewogen. Von einer 1 Gew.-%-igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

**Beispiel 3**

**Wirkung gegen Lucilia sericata**

Zu 9 ml eines Zuchtmediums wird bei 50°C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

**Beispiel 4**

**Wirkung gegen Aëdes aegypti**

Auf die Oberfläche von 150 ml Wasser, das sich in einem Behälter befindet, wird so viel einer 0,1 Gew.-%-igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

**Beispiel 5**

**Insektizide Frassgift-Wirkung**

Ca. 25 cm hohe eingetopfte Baumwollpflanzen werden mit wässrigen Wirkstoffemulsionen besprüht, die den jeweiligen Wirkstoff in Konzentrationen von 0,2 ppm bis 400 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und 60 % relativer Luftfeuchtigkeit durchgeführt. Nach 120 Stunden wird die %-Mortalität der Test-Insekten bestimmt.

Gegen Spodoptera Larven zeigen im obigen Test erfindungsgemässe Verbindungen in folgenden Konzentrationen 80 - 100 %-ige Wirksamkeit (Mortalität):

| Verbindung Nr. | Wirkstoffkonzentration |
|---|---|
| 17 | 0,2 ppm |
| 1, 3, 9, 15, 18, 24, 27 | 0,75 ppm |
| 8, 16, 19, 23, 29, 30, 31, 32, 33, 34, 36 | 3 ppm |
| 21, 22, 25, 28, 39, 40 | 12,5 ppm |
| 38 | 50 ppm |
| 2, 5, 6, 7, 41 | 100 ppm |
| 11, 20, 37 | 400 ppm |

Gegen Heliothis Larven zeigen im obigen Test erfindungsgemässe Verbindungen in folgenden Konzentrationen 80 - 100 %-ige Wirksamkeit (Mortalität):

| Verbindung Nr. | Wirkstoffkonzentration |
|---|---|
| 15, 28, 32, 36 | 12,5 ppm |
| 1, 8, 16, 17, 23, 24, 26, 29 | 50 ppm |
| 3, 7, 9, 18, 21, 25, 31, 39 | 100 ppm |
| 5, 27, 33, 40 | 200 ppm |
| 22, 41 | 400 ppm |

**Beispiel 6**

**Wirkung gegen Epilachna varivestis**

Etwa 15 - 20 cm hohe Phaseolus vulgaris-Pflanzen (Buschbohnen) werden mit wässrigen, den zu prüfenden Wirkstoff in Konzentrationen von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Über die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die % Mortalität bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Verbindungen der Formel I gemäss Beispiel 1 zeigen gute Wirkung im obigen Test.

**Beispiel 7**

**Ovizide Wirkung auf Spodoptera littoralis**

Entsprechende Mengenanteile einer benetzbaren pulverförmigen Formulierung, enthaltend 25 Gew.% des zu prüfenden

Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsionen werden eintägige Eigelege von Spodoptera auf Cellophan während drei Minuten eingetaucht und dann auf Rundfilter abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt.

Verbindungen Nr. 33 gemäss Beispiel 1 zeigt 80 - 100 %-ige Wirkung (Mortalität) in obigem Test.

**Beispiel 8**

**Wirkung auf Laspeyresia pomonella (Eier)**

Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet und die %-Mortalität bestimmt.

Verbindungen der Formel I gemäss Beispiel 1 zeigen gute Wirkung in obigem Test.

**Beispiel 9**

**Reproduktions-Beeinflussung von Anthonomus grandis**

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.-% des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss Beispiel 1 zeigen eine gute reproduktionsreduzierende Wirkung im obigen Test.

**Beispiel 10**

**Wirkung gegen Tetranychus cinnabarinus**

Die Primärblätter von Phaseolus vulgaris-Pflanzen werden 16 Stunden vor dem Versuch auf akarizide Wirkung mit einem infestierten Blattstück aus einer Massenzucht von Tetranychus cinnabarinus (OP-tol.) belegt (die Toleranz bezieht sich auf die Verträglichkeit gegenüber Diazinon).

Die so behandelten infestierten Pflanzen werden mit einer Versuchslösung enthaltend 400, 200 und 12,5 ppm der zu prüfenden Verbindung bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet.

Man verwendet pro Konzentration eine Pflanze. Während des Versuchsverlaufs stehen die Pflanzen in Gewächshauskabinen bei 25°C.

Verbindung Nr. 26 gemäss dem Beispiel 1 zeigt bei 12,5 ppm nach 7 Tagen in diesem Versuch 80 - 100 %-ige Wirkung (Mortalität).

**Beispiel 11**

**Wirkung gegen Tetranychus urticae**

Phaseolus vulgaris Pflanzen werden 12 Stunden vor dem Test auf akarizide Wirkung mit einem infestierten Blattstück aus einer Massenzucht von Tetranychus urticae belegt. Die übergelaufenen beweglichen Stadien werden aus einem Chromatographiezerstäuber mit den emulgierten Testpräparaten derart besprüht, dass kein Ablaufen der Spritzbrühe

eintritt. Die verwendeten Emulsionszubereitungen weisen eine Wirkstoffkonzentration von 800 ppm auf. Nach zwei und 7 Tagen werden Larven, Adulte und Eier unter dem Binokular auf lebende und tote Individuen ausgewertet.

Während der "Haltezeit" stehen die behandelten Pflanzen in Gewächshauskabinen bei 25°C.

Verbindungen der Formel I gemäss Beispiel 1 zeigen im obigen Test gute Wirkung.

**Beispiel 12**

**Wirkung gegen Anthonomus grandis (Adulte)**

Zwei eingetopfte Baumwollpflanzen im 6-Blattstadium werden jeweils mit wässrigen benetzungsfähigen Emulsions-Zubereitungen enthaltend 200 bzw. 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Öffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindung Nr. 15 gemäss Beispiel 1 zeigt 80 - 100 %ige Wirkung (Mortalität) in obigem Test bei 200 ppm.

**Beispiel 13**

**Formulierungen für Wirkstoffe der Formel I resp. Kombinationen dieser Wirkstoffe mit andern Insektiziden oder Akariziden (% Gewichtsprozent)**

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7 - 8 Mol ÄO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrat | |
|---|---|
| Wirkstoff oder Wirkstoffkombination | 10 % |
| Octylphenolpolyäthylenglykoläther (4 - 5 Mol ÄO) | 3 % |
| Ca-Dodecylbenzolsulfonat | 3 % |
| Ricinusölpolyglykoläther (36 Mol ÄO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Äthylenglykol | 10 % |
| Nonylphenolpolyäthylenglykoläther (15 Mol ÄO) | 6 % |
| Na-Ligninsulfonat | 10 % |
| Carboxymethylcellulose | 1 % |
| 37 %-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Patentansprüche** für die Vertragsstaaten: BE, CH, DE, FR, IT, LI, NL

1. Verbindung der Formel I

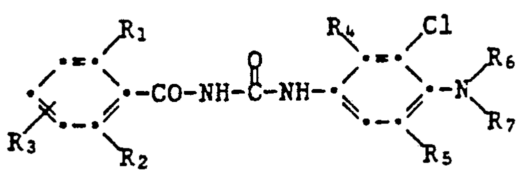

worin

$R_1$ Wasserstoff, Fluor, Chlor, Brom, $CF_3$ oder $C_1$-$C_4$-Alkoxy;
$R_2$ Fluor, Chlor, Brom, $C_1$-$C_4$-Alkyl, $CF_3$ oder $C_1$-$C_4$-Alkoxy;
$R_3$ Wasserstoff, Fluor, Chlor, Brom, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_6$-Alkyl, $C_3$-$C_5$-Alkenyl, $C_3$-$C_5$-Alkynyl oder mit 1 bis 8 Fluor- oder Chloratomen, Methoxy oder Äthoxy substituiertes $C_1$-$C_4$-Alkyl; oder
$R_6$ und $R_7$ zusammen mit dem N-Atom, an das sie gebunden sind, den

Rest 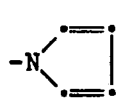 bedeuten.

2. Verbindung der Formel I gemäss Anspruch 1, dadurch gekennzeichnet, dass

$R_1$ Wasserstoff, Fluor, Chlor, Brom, $CF_3$ oder $C_1$-$C_4$-Alkoxy;
$R_2$ Fluor, Chlor, Brom, $C_1$-$C_4$-Alkyl, $CF_3$ oder $C_1$-$C_4$-Alkoxy;
$R_3$ Wasserstoff, Fluor, Chlor, Brom, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_6$-Alkyl, $C_3$-$C_5$-Alkenyl, $C_3$-$C_5$-Alkynyl oder mit 1 bis 8 Fluor- oder Chloratomen, Methoxy oder Äthoxy substituiertes $C_1$-$C_4$-Alkyl;

bedeuten.

3. Verbindung der Formel I gemäss Anspruch 1 oder 2, dadurch gekennzeichnet, dass

$R_1$ Wasserstoff, Fluor, Chlor, $CF_3$, Methoxy oder Äthoxy;
$R_2$ Fluor, Chlor, $C_1$-$C_3$-Alkyl, $CF_3$ oder Methoxy;
$R_3$ Wasserstoff, Fluor, Chlor, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_4$-Alkyl, Allyl, Propargyl oder mit 1 bis 7 Fluor- oder Chloratomen oder Methoxy substituiertes $C_1$-$C_3$-Alkyl;

bedeuten.

4. Verbindung der Formel I gemäss Anspruch 3, dadurch gekennzeichnet, dass

$R_1$ Wasserstoff, Fluor, Chlor oder Methoxy;
$R_2$ Fluor, Chlor, Methyl oder Methoxy;
$R_3$ Wasserstoff oder Fluor;
$R_4$ Fluor oder Chlor;
$R_5$ Wasserstoff oder Chlor;
$R_6$ und $R_7$ $C_1$-$C_4$-Alkyl

bedeuten.

5. Verbindung der Formel I gemäss einem der Ansprüche 1 bis 4, dadurch gekennzeichnet, dass Methyl und $R_7$ $C_1$-$C_4$-Alkyl bedeuten.

6. Verbindung der Formel I gemäss einem der Ansprüche 1 bis 5, dadurch gekennzeichnet, dass $R_4$ Fluor und $R_5$ Wasserstoff oder Chlor bedeuten.

7. Verbindung gemäss Anspruch 4 der Formel

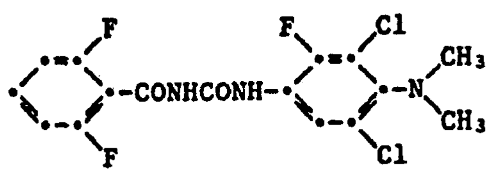

8. Verbindung gemäss Anspruch 4 der Formel

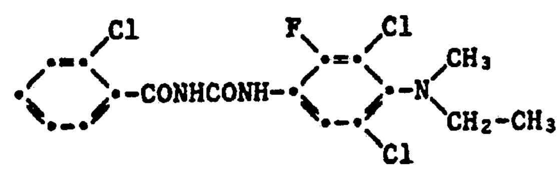

9. Verbindung gemäss Anspruch 4 der Formel

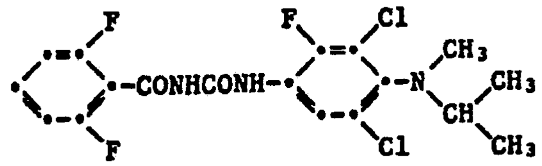

10. Verbindung gemäss Anspruch 4 der Formel

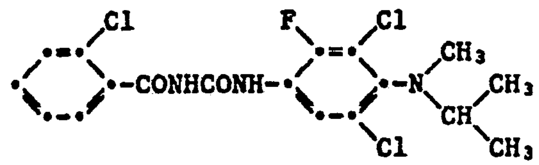

11. Verbindung gemäss Anspruch 4 der Formel

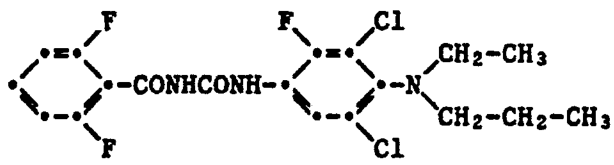

12. Verbindung gemäss Anspruch 4 der Formel

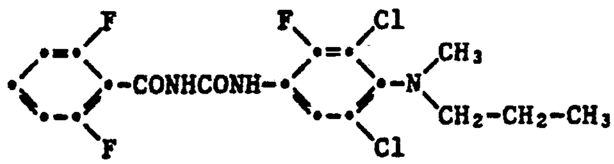

13. Verbindung gemäss Anspruch 4 der Formel

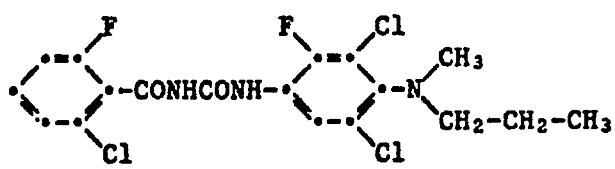

14. Verhindung gemäss Anspruch 4 der Formel

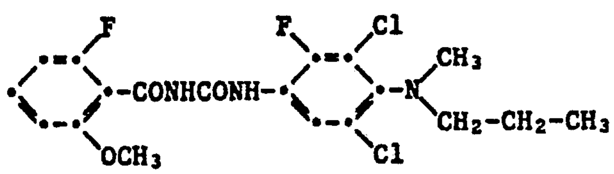

15. Verbindung gemäss Anspruch 4 der Formel

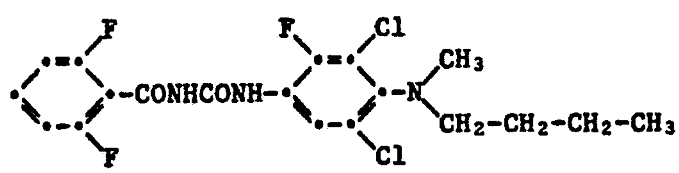

16. Verbindung gemäss Anspruch 4 der Formel

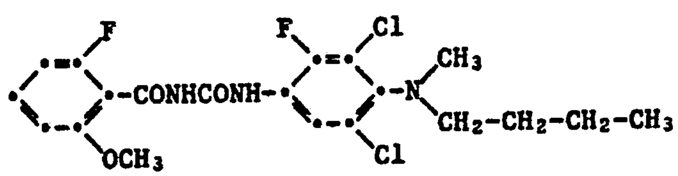

17. Verfahren zur Herstellung einer Verbindung gemäss den Ansprüchen 1 bis 16, dadurch gekennzeichnet, dass man a) eine Verbindung der Formel II

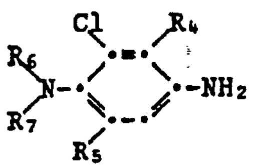

(II)

mit einer Verbindung der Formel III

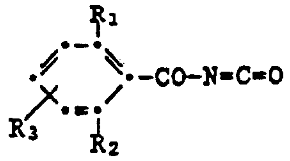

(III)

oder

b) eine Verbindung der Formel IV

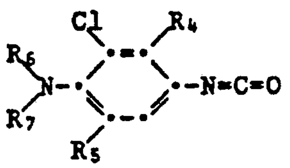

(IV)

mit einer Verbindung der Formel V

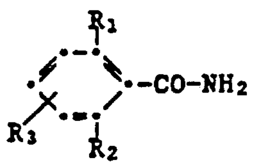

(V),

oder

c) eine Verbindung der Formel II mit einer Verbindung der Formel VI

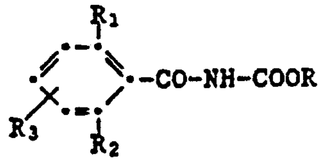

(VI)

umsetzt, wobei in den Formeln II bis VI die Reste $R_1$ bis $R_7$ die in den Ansprüchen 1 bis 6 angegebenen Bedeutungen haben und R für einen $C_1$-$C_8$-Alkylrest, der gegebenenfalls mit Halogen substituiert ist, steht.

18. Verbindung der Formel II

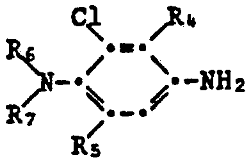

(II),

worin

$R_4$ bis $R_7$ die gemäss den Ansprüchen 1 bis 6 angegebenen Bedeutungen haben.

19. Schädlingsbekämpfungsmittel, welches als aktive Komponente eine Verbindung gemäss einem der Ansprüche 1 bis 16 zusammen mit geeigneten Trägern und/oder anderen Zuschlagstoffen enthält.

20. Verwendung einer Verbindung gemäss einem der Ansprüche 1 bis 16 zur Bekämpfung von Insekten und Vertretern der Ordnung Akarina.

21. Verwendung gemäss Anspruch 20 zur Bekämpfung larvaler Stadien pflanzenschädigender fressender Insekten.

22. Verfahren zur Bekämpfung von Insekten und Vertretern der Ordnung Akarina, dadurch gekennzeichnet, dass man die Insekten bzw. deren verschiedene Entwicklungsstadien oder deren Aufenthaltsort mit einer pestizid wirksamen Menge einer Verbindung der Formel I gemäss einem der Ansprüche 1 bis 16 oder mit einem Mittel enthaltend neben Zusatz- und Trägerstoffen eine pestizid wirksame Menge dieser Verbindung, in Kontakt bringt oder behandelt.

**Patentansprüche** für den Vertragsstaat: AT

1. Verfahren zur Herstellung einer Verbindung der Formel I

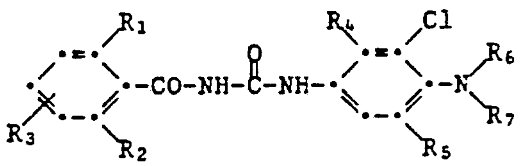

(I),

worin

$R_1$ Wasserstoff, Fluor, Chlor, Brom, $CF_3$ oder $C_1$-$C_4$-Alkoxy;
$R_2$ Fluor, Chlor, Brom, $C_1$-$C_4$-Alkyl, $CF_3$ oder $C_1$-$C_4$-Alkoxy;
$R_3$ Wasserstoff, Fluor, Chlor, Brom, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_6$-Alkyl, $C_3$-$C_5$-Alkenyl, $C_3$-$C_5$-Alkynyl oder mit 1 bis 8 Fluor- oder Chloratomen, Methoxy oder Äthoxy substituiertes $C_1$-$C_4$-Alkyl; oder
$R_6$ und $R_7$ zusammen mit dem N-Atom, an das sie gebunden sind, den

Rest 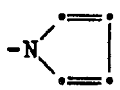 bedeuten, dadurch gekennzeichnet, dass man

a) eine Verbindung der Formel II

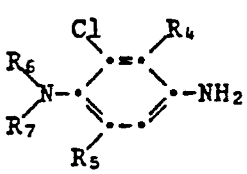

(II)

mit einer Verbindung der Formel III

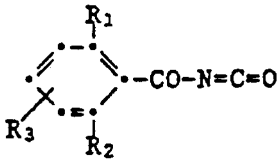

(III)

oder
b) eine Verbindung der Formel IV

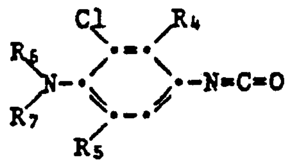

(IV)

mit einer Verbindung der Formel V

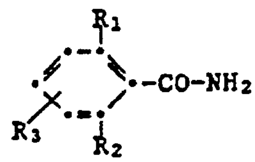

(V)

oder
c) eine Verbindung der Formel II mit einer Verbindung der Formel VI

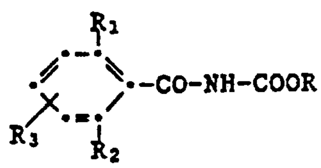

(VI)

umsetzt, wobei in den Formeln II bis VI die Reste $R_1$ bis $R_7$ die oben angegebenen Bedeutungen haben und R für einen $C_1$-$C_8$-Alkylrest, der gegebenenfalls mit Halogen substituiert ist, steht.

2. Verfahren nach Anspruch 1 zur Herstellung einer Verbindung der Formel I, worin

$R_1$ Wasserstoff, Fluor, Chlor, Brom, $CF_3$ oder $C_1$-$C_4$-Alkoxy;
$R_2$ Fluor, Chlor, Brom, $C_1$-$C_4$-Alkyl, $CF_3$ oder $C_1$-$C_4$-Alkoxy;
$R_3$ Wasserstoff, Fluor, Chlor, Brom, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_6$-Alkyl, $C_3$-$C_5$-Alkenyl, $C_3$-$C_5$-Alkynyl oder mit 1 bis 8 Fluor- oder Chloratomen, Methoxy oder Äthoxy substituiertes $C_1$-$C_4$-Alkyl;
bedeuten.

3. Verfahren gemäss Anspruch 1 oder 2, zur Herstellung einer Verbindung der Formel I, worin

$R_1$ Wasserstoff, Fluor, Chlor, $CF_3$, Methoxy oder Äthoxy;
$R_2$ Fluor, Chlor, $C_1$-$C_3$-Alkyl, $CF_3$ oder Methoxy;
$R_3$ Wasserstoff, Fluor, Chlor, $CF_3$ oder Methyl;
$R_4$ Fluor oder Chlor;
$R_5$ Wasserstoff, Fluor oder Chlor;
$R_6$ und $R_7$ unabhängig voneinander $C_1$-$C_4$-Alkyl, Allyl, Propargyl oder mit 1 bis 7 Fluor- oder Chloratomen oder Methoxy substituiertes $C_1$-$C_3$-Alkyl;
bedeuten.

4. Verfahren gemäss Anspruch 3, zur Herstellung einer Verbindung der Formel I, worin

$R_1$ Wasserstoff, Fluor, Chlor oder Methoxy;
$R_2$ Fluor, Chlor, Methyl oder Methoxy;
$R_3$ Wasserstoff oder Fluor;
$R_4$ Fluor oder Chlor;
$R_5$ Wasserstoff oder Chlor;
$R_6$ und $R_7$ $C_1$-$C_4$-Alkyl
bedeuten.

5. Verfahren gemäss einem der Ansprüche 1 bis 4, zur Herstellung einer Verbindung der Formel I, worin $R_6$ Methyl und $R_7$ $C_1$-$C_4$-Alkyl bedeuten.

6. Verfahren gemäss einem der Ansprüche 1 bis 5, zur Herstellung einer Verbindung der Formel I, worin $R_4$ Fluor und $R_5$ Wasserstoff oder Chlor bedeuten.

7. Verfahren gemäss Anspruch 4 zur Herstellung der Verbindung der Formel

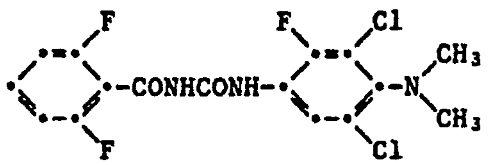

8. Verfahren gemäss Anspruch 4 zur Herstellung der Verbindung der Formel

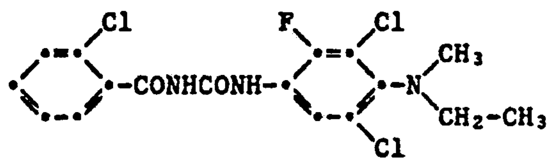

9. Verfahren gemäss Anspruch 4 zur Herstellung der Verbindung der Formel

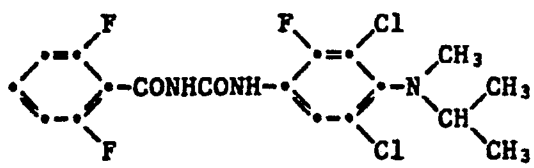

10. Verfahren gemäss Anspruch 4 zur Herstellung der Verbindung der Formel

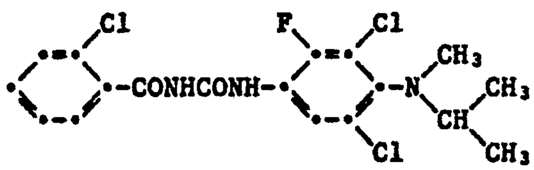

11. Verfahren gemäss Anspruch 4 zur Herstellung der Verbindung der Formel

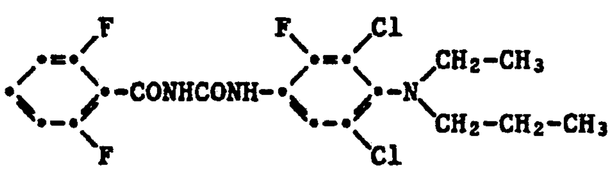

12. Verfahren gemäss Anspruch 4 zur Herstellung der Verbindung der Formel

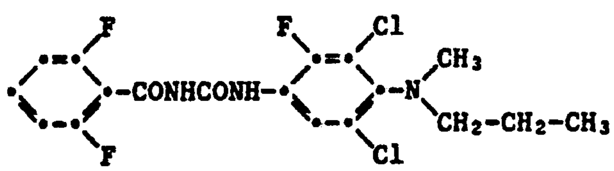

13. Verfahren gemäss Anspruch 4 zur Herstellung der Verbindung der Formel

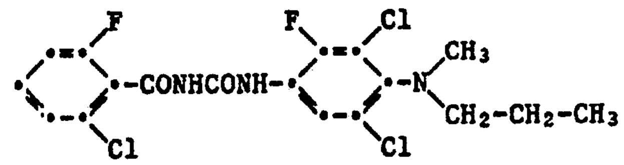

14. Verfahren gemäss Anspruch 4 zur Herstellung der Verbindung der Formel

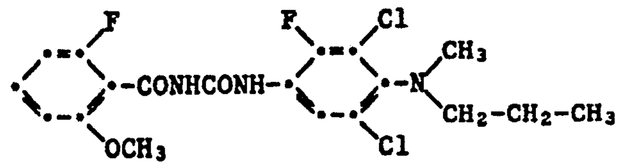

15. Verfahren gemäss Anspruch 4 zur Herstellung der Verbindung der Formel

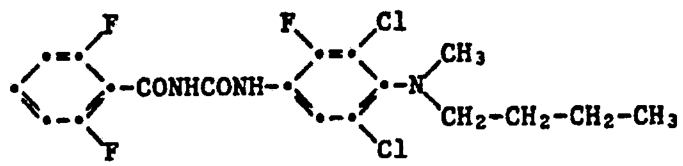

16. Verfahren gemäss Anspruch 4 zur Herstellung der Verbindung der Formel

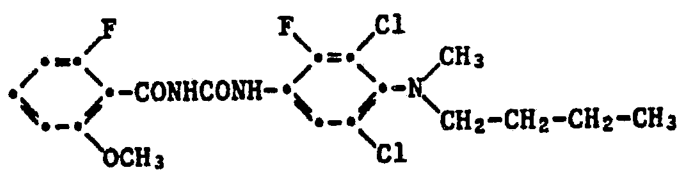

17. Schädlingsbekämpfungsmittel, welches als aktive Komponente eine Verbindung gemäss einem der Ansprüche 1 bis 16 zusammen mit geeigneten Trägern und/oder anderen Zuschlagstoffen enthält.

18. Verwendung einer Verbindung gemäss einem der Ansprüche 1 bis 16 zur Bekämpfung von Insekten und Vertretern der Ordnung Akarina.

19. Verwendung gemäss Anspruch 18 zur Bekämpfung larvaler Stadien pflanzenschädigender fressender Insekten.

20. Verfahren zur Bekämpfung von Insekten und Vertretern der Ordnung Akarina, dadurch gekennzeichnet, dass man die Insekten bzw. deren verschiedene Entwicklungsstadien oder deren Aufenthaltsort mit einer pestizid wirksamen Menge einer Verbindung der Formel I gemäss einem der Ansprüche 1 bis I6 oder mit einem Mittel enthaltend neben Zusatz- und Trägerstoffen eine pestizid wirksame Menge dieser Verbindung, in Kontakt bringt oder behandelt.

**Claims** for the Contracting States: BE, CH, DE, FR, IT, LI, NL

1. A compound of formula I

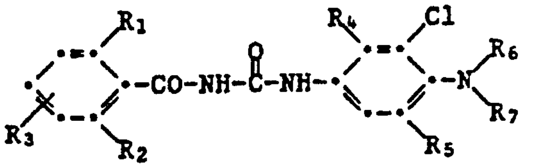

(I),

wherein

$R_1$ is hydrogen, fluoro, chloro, bromo, $CF_3$ or $C_1$-$C_4$alkoxy,
$R_2$ is fluoro, chloro, bromo, $C_1$-$C_4$alkyl, $CF_3$ or $C_1$-$C_4$alkoxy,
$R_3$ is hydrogen, fluoro, chloro, bromo, $CF_3$ or methyl,
$R_4$ is fluoro or chloro,
$R_5$ is hydrogen, fluoro or chloro,
$R_6$ and $R_7$ are each independently of the other $C_1$-$C_6$alkyl, $C_3$-$C_5$alkenyl, $C_3$-$C_5$alkynyl, or $C_1$-$C_4$alkyl which is substituted by 1 to 8 fluorine or chlorine atoms or by methoxy or ethoxy; or
$R_6$ and $R_7$, together with the nitrogen atom to which they are attached, are the radical

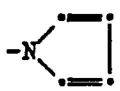

2. A compound of formula I according to claim 1, wherein

$R_1$ is hydrogen, fluoro, chloro, bromo, $CF_3$ or $C_1$-$C_4$alkoxy,
$R_2$ is fluoro, chloro, bromo, $C_1$-$C_4$alkyl, $CF_3$ or $C_1$-$C_4$alkoxy,
$R_3$ is hydrogen, fluoro, chloro, bromo, $CF_3$ or methyl,
$R_4$ is fluoro or chloro,
$R_5$ is hydrogen, fluoro or chloro,
$R_6$ and $R_7$ are each independently of the other $C_1$-$C_6$alkyl, $C_3$-$C_5$alkenyl, $C_3$-$C_5$alkynyl, or $C_1$-$C_4$alkyl which is substituted by 1 to 8 fluorine or chlorine atoms or by methoxy or ethoxy.

3. A compound of formula I according to either claim 1 or claim 2, wherein

$R_1$ is hydrogen, fluoro, chloro, $CF_3$, methoxy or ethoxy,
$R_2$ is fluoro, chloro, $C_1$-$C_3$alkyl, $CF_3$ or methoxy,
$R_3$ is hydrogen, fluoro, chloro, $CF_3$ or methyl,
$R_4$ is fluoro or chloro,
$R_5$ is hydrogen, fluoro or chloro, and
$R_6$ and $R_7$ are each independently of the other $C_1$-$C_4$alkyl, allyl, propargyl, or $C_1$-$C_3$alkyl which is substituted by 1 to 7 fluorine or chlorine atoms or by methoxy.

4. A compound of formula I according to claim 3, wherein

$R_1$ is hydrogen, fluoro, chloro or methoxy,
$R_2$ is fluoro, chloro, methyl or methoxy,
$R_3$ is hydrogen or fluoro,
$R_4$ is fluoro or chloro,
$R_5$ is hydrogen or chloro, and
$R_6$ and $R_7$ are $C_1$-$C_4$alkyl.

5. A compound of formula I according to any one of claims 1 to 4, wherein $R_6$ is methyl and $R_7$ is $C_1$-$C_4$alkyl.

6. A compound of formula I according to any one of claims 1 to 5, wherein $R_4$ is fluoro and $R_5$ is hydrogen or chloro.

7. A compound according to claim 4 of formula

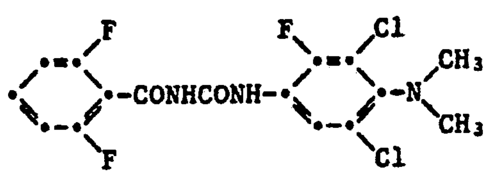

8. A compound according to claim 4 of formula

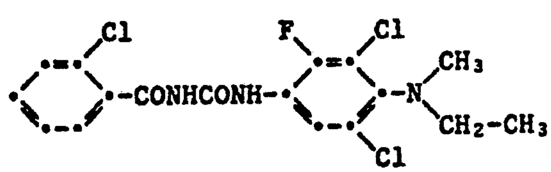

9. A compound according to claim 4 of formula

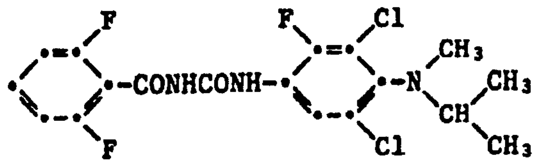

10. A compound according to claim 4 of formula

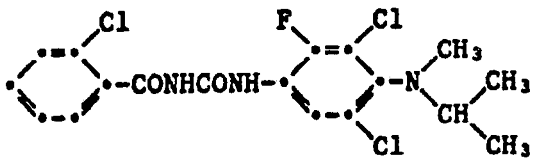

11. A compound according to claim 4 of formula

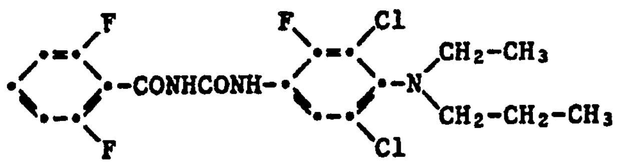

12. A compound according to claim 4 of formula

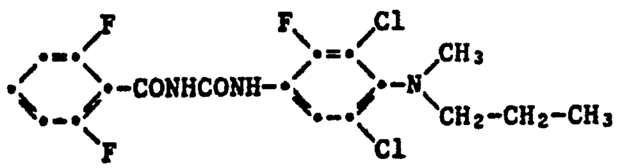

13. A compound according to claim 4 of formula

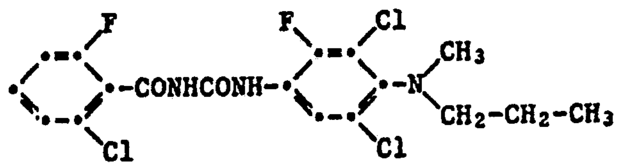

14. A compound according to claim 4 of formula

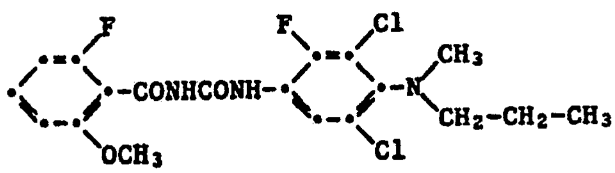

15. A compound according to claim 4 of formula

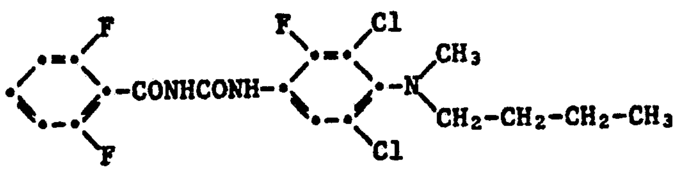

16. A compound according to claim 4 of formula

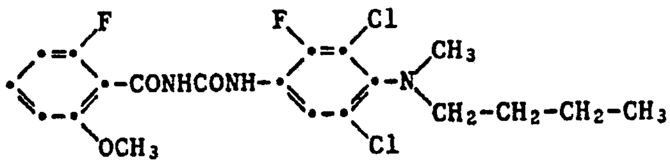

17. A process for the preparation of a compound according to any one of claims 1 to 16, which comprises

a) reacting a compound of formula II

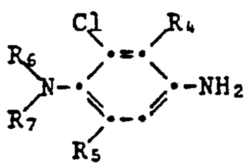

(II)

with a compound of formula III

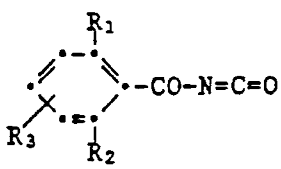

(III)

or

b) reacting a compound of formula IV

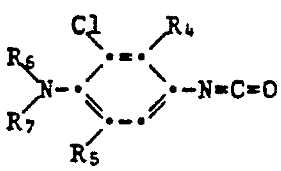

(IV)

with a compound of formula V

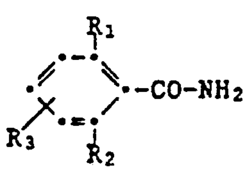

(V),

or

c) reacting a compound of formula II with a compound of formula VI

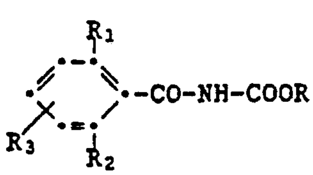

(VI),

in which formulae II to VI above $R_1$ to $R_7$ are as defined in claims 1 to 6 and R is a $C_1$-$C_8$alkyl radical which may be

substituted by halogen.

18. A compound of formula II

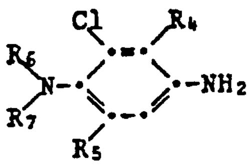

(II)

wherein $R_4$ to $R_7$ are as defined in claims 1 to 6.

19. A pesticidal composition which contains as active component a compound as claimed in any one of claims 1 to 16, together with suitable carriers and/or other adjuvants.

20. Use of a compound according to any one of claims 1 to 16 for controlling insects and representatives of the order Acarina.

21. Use according to claim 20 for controlling larval insect stages of plant-destructive feeding insects.

22. A method of controlling insects and representatives of the order Acarina, which comprises treating or contacting said insects, their various development stages or the locus thereof, with a pesticidally effective amount of a compound of formula I according to any one of claims 1 to 16, or with a composition which contains a pesticidally effective amount of such a compound, together with adjuvants and carriers suitable therefor.

**Claims** for Contracting State: AT

1. A process for the preparation of a compound of formula I

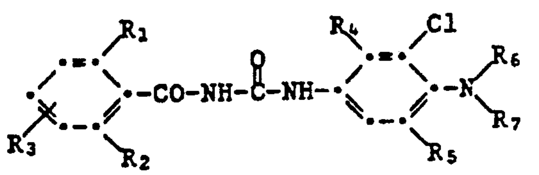

(I),

wherein

$R_1$ is hydrogen, fluoro, chloro, bromo, $CF_3$ or $C_1$-$C_4$alkoxy,
$R_2$ is fluoro, chloro, bromo, $C_1$-$C_4$alkyl, $CF_3$ or $C_1$-$C_4$alkoxy,
$R_3$ is hydrogen, fluoro, chloro, bromo, $CF_3$ or methyl,
$R_4$ is fluoro or chloro,
$R_5$ is hydrogen, fluoro or chloro,
$R_6$ and $R_7$ are each independently of the other $C_1$-$C_6$alkyl, $C_3$-$C_5$alkenyl, $C_3$-$C_5$alkynyl, or $C_1$-$C_4$alkyl which is substituted by 1 to 8 fluorine or chlorine atoms or by methoxy or ethoxy; or
$R_6$ and $R_7$, together with the nitrogen atom to which they are attached, are the radical

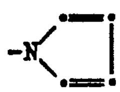

which process comprises

a) reacting a compound of formula II

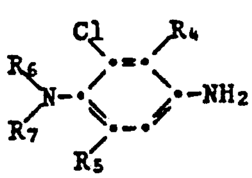

(II)

with a compound of formula III

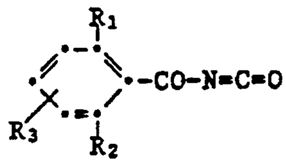

(III)

or

b) reacting a compound of formula IV

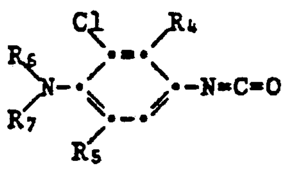

(IV)

with a compound of formula V

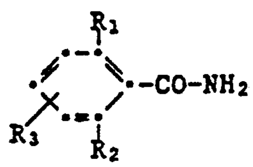

(V),

or

c) reacting a compound of formula II with a compound of formula VI

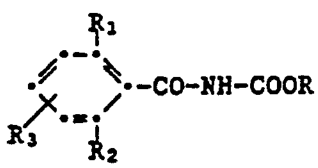

(VI).

in which formulae II to VI above $R_1$ to $R_7$ are as defined in claims 1 to 6 and R is a $C_1$-$C_8$alkyl radical which may be substituted by halogen.

2. A process according to claim 1 for the preparation of a compound of formula I, wherein

$R_1$ is hydrogen, fluoro, chloro, bromo, $CF_3$ or $C_1$-$C_4$alkoxy,
$R_2$ is fluoro, chloro, bromo, $C_1$-$C_4$alkyl, $CF_3$ or $C_1$-$C_4$alkoxy,
$R_3$ is hydrogen, fluoro, chloro, bromo, $CF_3$ or methyl,
$R_4$ is fluoro or chloro,
$R_5$ is hydrogen, fluoro or chloro,
$R_6$ and $R_7$ are each independently of the other $C_1$-$C_6$alkyl, $C_3$-$C_5$alkenyl, $C_3$-$C_5$alkynyl, or $C_1$-$C_4$alkyl which is substituted by 1 to 8 fluorine or chlorine atoms or by methoxy or ethoxy.

3. A process according to claim 1 or claim 2 for the preparation of a compound of formula I, wherein

$R_1$ is hydrogen, fluoro, chloro, $CF_3$, methoxy or ethoxy,
$R_2$ is fluoro, chloro, $C_1$-$C_3$alkyl, $CF_3$ or methoxy,
$R_3$ is hydrogen, fluoro, chloro, $CF_3$ or methyl,
$R_4$ is fluoro or chloro,
$R_5$ is hydrogen, fluoro or chloro, and
$R_6$ and $R_7$ are each independently of the other $C_1$-$C_4$alkyl, allyl, propargyl, or $C_1$-$C_3$alkyl which is substituted by 1 to 7 fluorine or chlorine atoms or by methoxy.

4. A process according to claim 3 for the preparation of a compound of formula I, wherein

$R_1$ is hydrogen, fluoro, chloro or methoxy,
$R_2$ is fluoro, chloro, methyl or methoxy,
$R_3$ is hydrogen or fluoro,
$R_4$ is fluoro or chloro,
$R_5$ is hydrogen or chloro, and
$R_6$ and $R_7$ are $C_1$-$C_4$alkyl.

5. A process according to any one of claims 1 to 4 for the preparation of a compound of formula I, wherein $R_6$ is methyl and $R_7$ is $C_1$-$C_4$alkyl.

6. A process according to any one of claims 1 to 5 for the preparation of a compound of formula I, wherein $R_4$ is fluoro and $R_5$ is hydrogen or chloro.

7. A process according to claim 4 for the preparation of the compound of formula

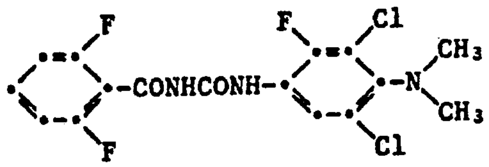

8. A process according to claim 4 for the preparation of the compound of formula

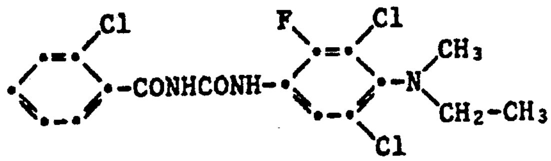

9. A process according to claim 4 for the preparation of the compound of formula

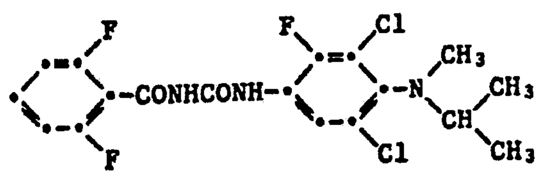

10. A process according to claim 4 for the preparation of the compound of formula

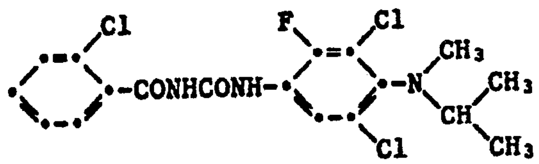

11. A process according to claim 4 for the preparation of the compound of formula

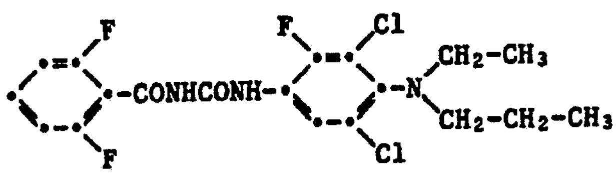

12. A process according to claim 4 for the preparation of the compound of formula

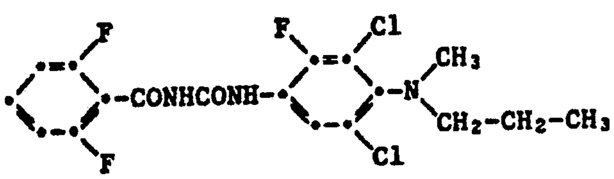

13. A process according to claim 4 for the preparation of the compound of formula

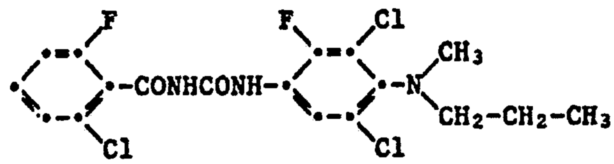

14. A process according to claim 4 for the preparation of the compound of formula

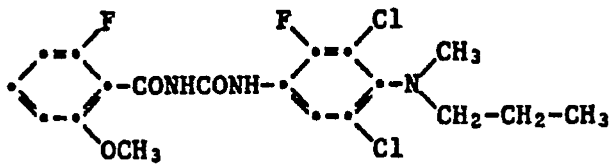

15. A process according to claim 4 for the preparation of the compound of formula

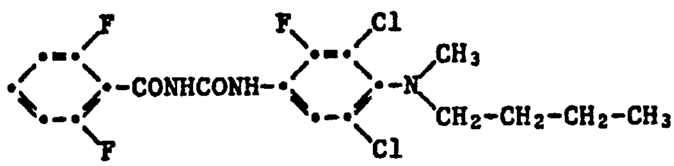

16. A process according to claim 4 for the preparation of the compound of formula

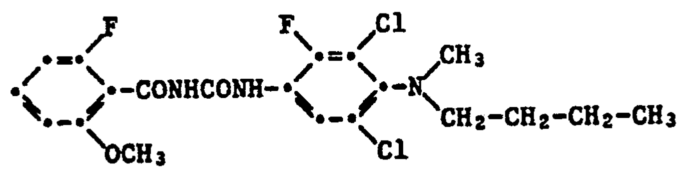

17. A pesticidal composition which contains as active component a compound as claimed in any one of claims 1 to 16, together with suitable carriers and/or other adjuvants.

18. Use of a compound according to any one of claims 1 to 16 for controlling insects and representatives of the order Acarina.

19. Use according to claim 18 for controlling larval insect stages of plant-destructive feeding insects.

20. A method of controlling insects and representatives of the order Acarina, which comprises treating or contacting said insects, their various development stages or the locus thereof, with a pesticidally effective amount of a compound of formula I according to any one of claims 1 to 16, or with a composition which contains a pesticidally effective amount of such a compound, together with adjuvants and carriers suitable therefor.

**Revendications** pour les Etats Contracants: BE, CH, DE, FR, IT, LI, NL

1. Composé de formule

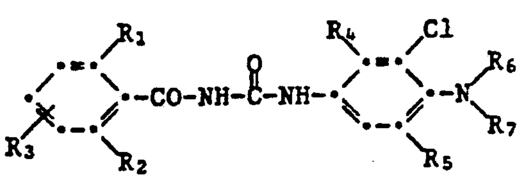

(I),

où

$R_1$ représente un hydrogène, fluor, chlore, brome, $CF_3$ ou alcoxy en $C_1$ à $C_4$;
$R_2$ représente un fluor, chlore, brome, alcoyle en $C_1$ à $C_4$, $CF_3$ ou alcoxy en $C_1$ à $C_4$;
$R_3$ représente un hydrogène, fluor, chlore, brome, $CF_3$ ou méthyle;
$R_4$ représente un fluor ou un chlore;
$R_5$ représente un hydrogène, fluor ou chlore;
$R_6$ et $R_7$ représentent indépendamment l'un de l'autre un alcoyle en $C_6$ à $C_7$, alcényle en $C_3$ à $C_5$, alcynyle en $C_3$ à $C_5$ ou alcoyle en $C_1$ à $C_4$ substitué par de 1 à 8 atomes de fluor ou de chlore, un méthoxy ou éthoxy; ou
$R_6$ et $R_7$ représentent ensemble avec l'atome de N auquel ils sont liés, le radical

2. Composé de formule I selon la revendication 1, caractérisé en ce que

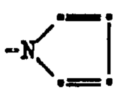

$R_1$ représente un hydrogène, fluor, chlore, brome, $CF_3$ ou alcoxy en $C_1$ à $C_4$;
$R_2$ représente un fluor, chlore, brome, alcoyle en $C_1$ à $C_4$, $CF_3$ ou alcoxy en $C_1$ à $C_4$;
$R_3$ représente un hydrogène, fluor, chlore, brome, $CF_3$ ou méthyle;
$R_4$ représente un fluor ou un chlore;
$R_5$ représente un hydrogène, fluor ou chlore;
$R_6$ et $R_7$ représentent indépendamment l'un de l'autre un alcoyle en $C_1$ à $C_6$, un alcényle en $C_3$ à $C_5$, un alcynyle en $C_3$ à $C_5$ ou un alcoyle en $C_1$ à $C_4$ substitué par de 1 à 8 atomes de fluor ou de chlore, un méthoxy ou un éthoxy.

3. Composé de formule I selon la revendication 1 ou 2, caractérisé en ce que

$R_1$ représente un hydrogène, fluor, chlore, $CF_3$, méthoxy ou éthoxy;
$R_2$ représente un fluor, chlore, alcoyle en $C_1$ à $C_3$, $CF_3$ ou méthoxy;
$R_3$ représente un hydrogène, fluor, chlore, $CF_3$ ou méthyle;
$R_4$ représente un fluor ou un chlore;
$R_5$ représente un hydrogène, fluor ou chlore;
$R_6$ et $R_7$ représentent indépendamment l'un de l'autre un alcoyle en $C_1$ à $C_4$, allyle, propargyle ou alcoyle en $C_1$ à $C_3$ substitué par de 1 à 7 atomes de fluor ou de chlore ou un méthoxy.

4. Composé de formule I selon la revendication 3, caractérisé en ce que

$R_1$ représente un hydrogène, fluor, chlore ou méthoxy;
$R_2$ représente un fluor, chlore, méthyle ou méthoxy;
$R_3$ représente un hydrogène ou un fluor;
$R_4$ représente un fluor ou un chlore;
$R_5$ représente un hydrogène ou un chlore;
$R_6$ et $R_7$ représentent un alcoyle en $C_1$ à $C_4$.

5. Composé de formule I selon l'une des revendications 1 à 4, caractérisé en ce que $R_6$ représente un méthyle et $R_7$ représente un alcoyle en $C_1$ à $C_4$.

6. Composé de formule I selon l'une des revendications 1 à 5, caractérisé en ce que $R_4$ représente un fluor et $R_5$ représente un hydrogène ou un chlore.

7. Composé selon la revendication 4 de formule

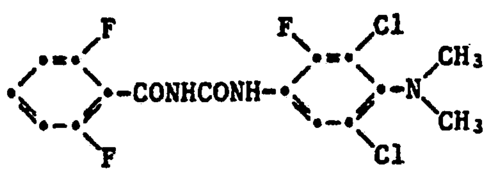

8. Composé selon la revendication 4 de formule

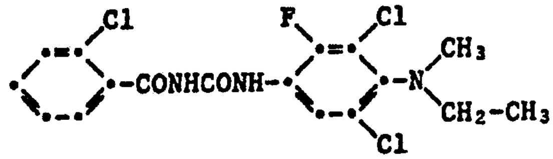

9. Composé selon la revendication 4 de formule

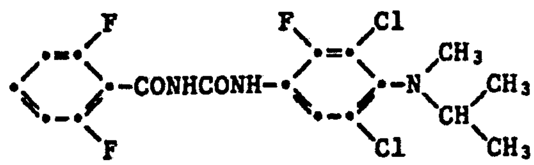

10. Composé selon la revendication 4 de formule

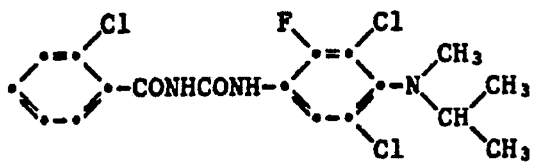

11. Composé selon la revendication 4 de formule

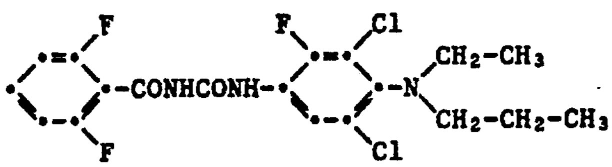

12. Composé selon la revendication 4 de formule

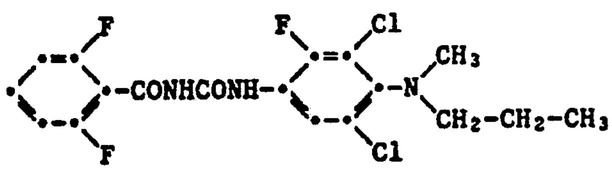

13. Composé selon la revendication 4 de formule

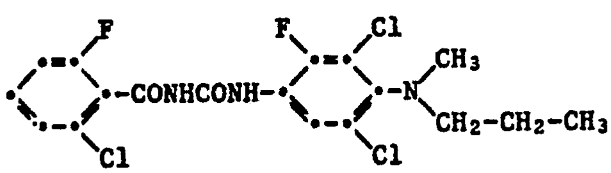

14. Composé selon la revendication 4 de formule

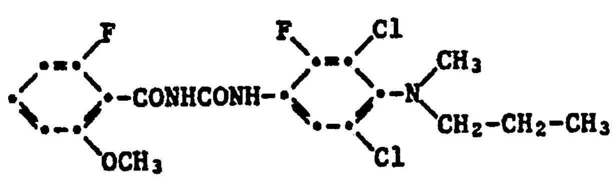

15. Composé selon la revendication 4 de formule

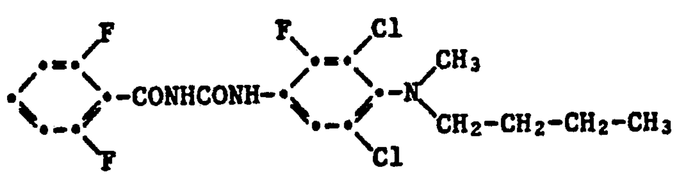

16. Composé selon la revendication 4 de formule

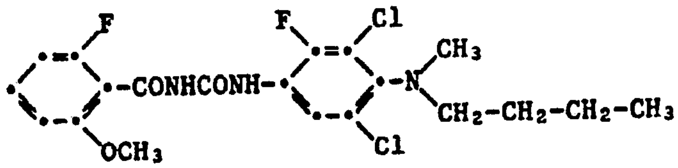

17. Procédé de préparation d'un composé selon les revendications 1 à 16, caractérisé en ce que l'on fait réagir

a) un composé de formule II

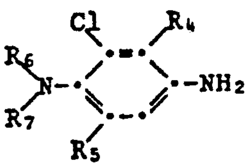

(II)

avec un composé se formule III

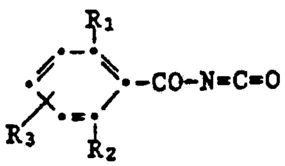

(III)

ou

b) un composé de formule IV

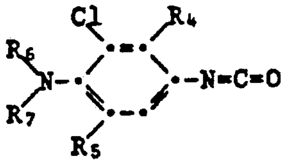

(IV)

avec un composé de formule V

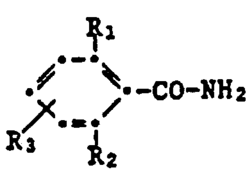

(V),

ou

c) un composé de formule II avec un composé de formule VI

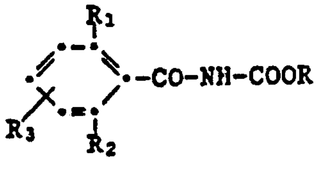

(VI)

où dans les formules II à VI les radicaux $R_1$ à $R_7$ ont les significations données dans les revendications 1 à 6 et R

représente un radical alcoyle en $C_1$ à $C_8$, qui est éventuellement substitué par un halogène.

18. Composé de formule II

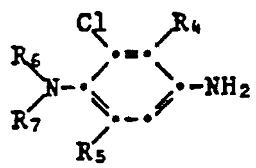

(II),

où
$R_4$ à $R_7$ ont les significations données selon les revendications 1 à 6.

19. Agent de lutte antiparasitaire qui contient comme composant actif un composé selon l'une des revendications 1 à 16 avec des supports et/ou autres additifs appropriés.

20. Application d'un composé selon l'une des revendications 1 à 16 à la lutte contre les insectes et les représentants de l'ordre des acariens.

21. Application selon la revendication 20 à la lutte contre les stades larvaires d'insectes phytophages.

22. Procédé de lutte contre les insectes et les représentants de l'ordre des acariens, caractérisé en ce qu'on met en contact ou traite les insectes, leurs différents stades de développement ou leur lieu de séjour avec une quantité à action pesticide d'un composé de formule I selon l'une des revendications 1 à 16 ou avec un agent contenant outre des additifs et supports une quantité à action pesticide de ce composé.

**Revendications** pour l'Etat Contracants: AT

1. Procédé de préparation d'un composé de formule I

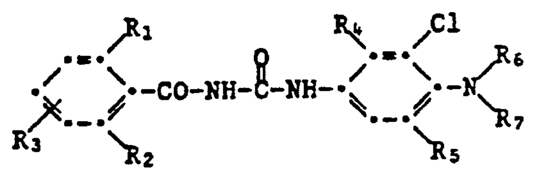

(I),

où

$R_1$ représente un hydrogène, fluor, chlore, brome, $CF_3$ ou alcoxy en $C_1$ à $C_4$;
$R_2$ représente un fluor, chlore, brome, alcoyle en $C_1$ à $C_4$, $CF_3$ ou alcoxy en $C_1$ à $C_4$;
$R_3$ représente un hydrogène, fluor, chlore, brome, $CF_3$ ou méthyle;
$R_4$ représente un fluor ou un chlore;
$R_5$ représente un hydrogène, fluor ou chlore;
$R_6$ et $R_7$ représentent indépendamment l'un de l'autre un alcoyle en $C_6$ à $C_7$, alcényle en $C_3$ à $C_5$, alcynyle en $C_3$ à $C_5$ ou alcoyle en $C_1$ à $C_4$ substitué par de 1 à 8 atomes de fluor ou de chlore, un méthoxy ou éthoxy; ou
$R_6$ et $R_7$ représentent ensemble avec l'atome de N auquel ils sont liés, le radical

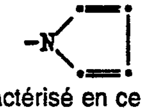

caractérisé en ce que l'on fait réagir

a) un composé de formule II

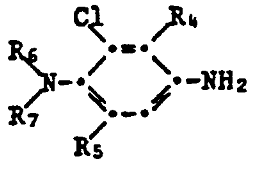

(II)

avec un composé se formule III

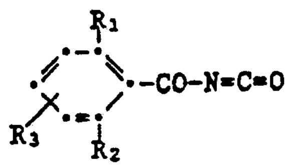

(III)

ou

b) un composé de formule IV

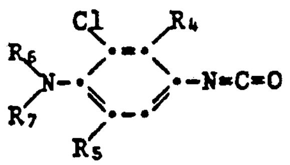

(IV)

avec un composé de formule V

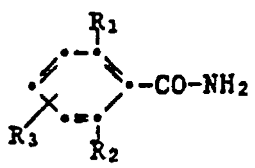

(V),

ou

c) un composé de formule II avec un composé de formule VI

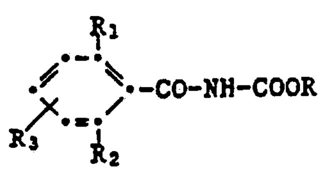

(VI)

où dans les formules II à VI les radicaux $R_1$ à $R_7$ ont les significations données dans les revendications 1 à 6 et R représente un radical alcoyle en $C_1$ à $C_8$, qui est éventuellement substitué par un halogène.

2. Procédé selon la revendication 1 de préparation d'un composé de formule I, où

$R_1$ représente un hydrogène, fluor, chlore, brome, $CF_3$ ou alcoxy en $C_1$ à $C_4$;
$R_2$ représente un fluor, chlore, brome, alcoyle en $C_1$ à $C_4$, $CF_3$ ou alcoxy en $C_1$ à $C_4$;
$R_3$ représente un hydrogène, fluor, chlore, brome, $CF_3$ ou méthyle;
$R_4$ représente un fluor ou un chlore;
$R_5$ représente un hydrogène, fluor ou chlore;
$R_6$ et $R_7$ représentent indépendamment l'un de l'autre un alcoyle en $C_1$ à $C_6$, alcényle en $C_3$ à $C_5$, alcynyle en $C_3$ à $C_5$ ou un alcoyle substitué par de 1 à 8 atomes de fluor ou de chlore, un méthoxy ou éthoxy.

3. Procédé selon la revendication 1 ou 2, de préparation d'un composé de formule I, où

$R_1$ représente un hydrogène, fluor, chlore, $CF_3$, méthoxy ou éthoxy;
$R_2$ représente un fluor, chlore, alcoyle en $C_1$ à $C_3$, $CF_3$ ou méthoxy;
$R_3$ représente un hydrogène, fluor, chlore, $CF_3$ ou méthyle;
$R_4$ représente un fluor ou un chlore;
$R_5$ représente un hydrogène, fluor ou chlore;
$R_6$ et $R_7$ représentent indépendamment l'un de l'autre un alcoyle en $C_1$ à $C_4$, allyle, propargyle ou alcoyle en $C_1$ à $C_3$ substitué par de 1 à 7 atomes de fluor ou de chlore ou un méthoxy.

4. Procédé selon la revendication 3 de préparation d'un composé de formule I, où

$R_1$ représente un hydrogène, fluor, chlore ou méthoxy,
$R_2$ représente un fluor, chlore, méthyle ou méthoxy,
$R_3$ représente un hydrogène ou un fluor;
$R_4$ représente un fluor ou un chlore;
$R_5$ représente un hydrogène ou un chlore;
$R_6$ et $R_7$ représentent un alcoyle en $C_1$ à $C_4$.

5. Procédé selon l'une des revendications 1 à 4, de préparation d'un composé de formule I, où $R_6$ représente un méthyle et $R_7$ représente un alcoyle en $C_1$ à $C_4$.

6. Procédé selon l'une des revendications 1 à 5, de préparation d'un composé de formule I, où $R_4$ représente un fluor et $R_5$ représente un hydrogène ou un chlore.

7. Procédé selon la revendication 4 de préparation du composé de formule

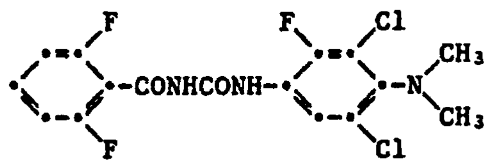

8. Procédé selon la revendication 4 de préparation du composé de formule

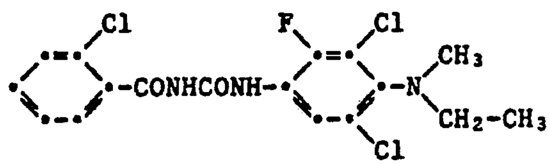

9. Procédé selon la revendication 4 de préparation du composé de formule

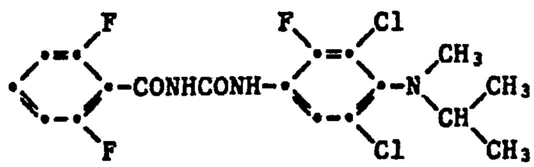

10. Procédé selon la revendication 4 de préparation du composé de formule

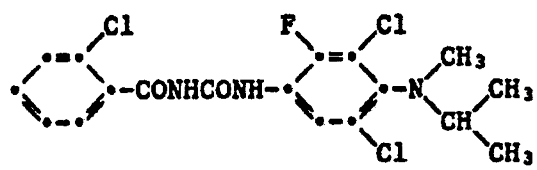

11. Procédé selon la revendication 4 de préparation du composé de formule

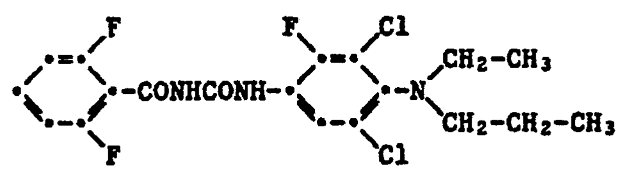

12. Procédé selon la revendication 4 de préparation du composé de formule

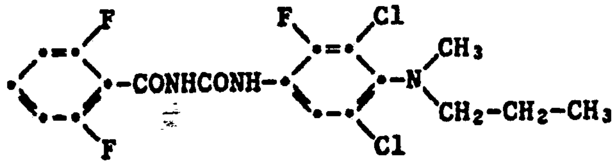

13. Procédé selon la revendication 4 de préparation du composé de formule

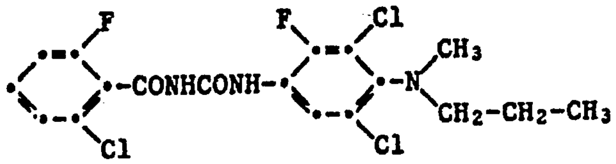

14. Procédé selon la revendication 4 de préparation du composé de formule

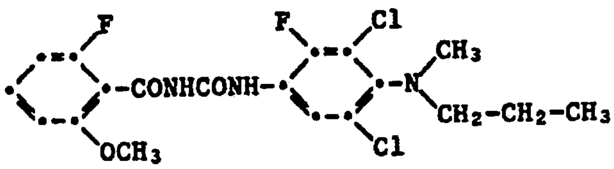

15. Procédé selon la revendication 4 de préparation du composé de formule

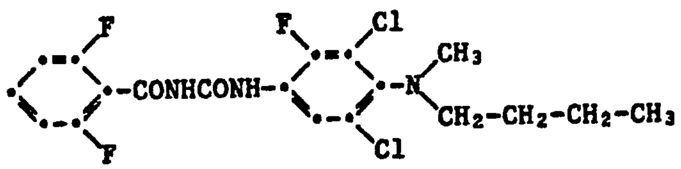

16. Procédé selon la revendication 4 de préparation du composé de formule

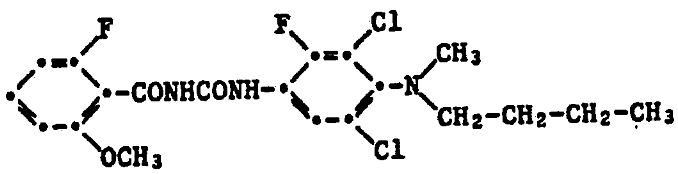

17. Agent de lutte antiparasitaire qui contient comme composant actif un composé selon l'une des revendications 1 à 16 avec des supports et/ou autres additifs appropriés.

18. Application d'un composé selon l'une des revendications 1 à 16 à la lutte contre les insectes et les représentants de l'ordre des acariens.

19. Application selon la revendication 18 à la lutte contre les stades larvaires d'insectes phytophages.

20. Procédé de lutte contre les insectes et les représentants de l'ordre des acariens, caractérisé en ce qu'on met en contact ou traite les insectes, leurs divers stades de développement ou leur lieu de séjour avec une quantité à action pesticide d'un composé de formule I selon l'une des revendications 1 à 16 ou avec un agent contenant outre des additifs et supports une quantité à action pesticide de ce composé.